Europäisches Patentamt

European Patent Office

Office européen des brevets

(11) Publication number: **0 403 649 B1**

## EUROPEAN PATENT SPECIFICATION

(45) Date of publication of patent specification: **15.02.95**　(51) Int. Cl.[6]: **A61F 2/24**

(21) Application number: **89902353.5**

(22) Date of filing: **14.12.88**

(86) International application number:
**PCT/SU88/00258**

(87) International publication number:
**WO 90/06734 (28.06.90 90/15)**

(54) **HEART VALVE PROSTHESIS.**

(43) Date of publication of application:
**27.12.90 Bulletin 90/52**

(45) Publication of the grant of the patent:
**15.02.95 Bulletin 95/07**

(84) Designated Contracting States:
**AT BE CH DE FR GB IT LI NL SE**

(56) References cited:
EP-A- 0 211 576　　EP-A- 0 327 790
GB-A- 1 447 871　　US-A- 4 274 437
US-A- 4 406 022　　US-A- 4 605 408
US-A- 4 692 165

(73) Proprietor: **MELNIKOV, Alexandr Petrovich**
**ul. Sosnovaya. 22-2-54**
**Kirovskaya obl**
**Kirovo-Chepetsk 613020 (SU)**

Proprietor: **KARTOSHKIN, Vyacheslav Mik-**
**hailovich**
**pr. Mira, 43e-39**
**Kirovskaya obl**
**Kirovo-Chepetsk 613020 (SU)**

Proprietor: **EVDOKIMOV, Sergei Vasilievich**
**ul. Chepetskaya, 20 70**
**Kirovskaya obl**
**Kirovo-Chepetsk 613020 (SU)**

Proprietor: **BROVKO, Miroslav Mikhailovich**
**ul. Entuziastov, 5-36**
**Kharkov, 310108 (SU)**

Proprietor: **GORSHKOV, Jury Vladimirovich**
**pr. Mira, 17-9**
**Kirovskaya obl**
**Kirovo-Chepetsk 613020 (SU)**

Proprietor: **PERIMOV, Jury Alexandrovich**
**pr. Mira, 21a-40**
**Kirovskaya obl**
**Kirovo-Chepetsk 613020 (SU)**

Proprietor: **MITROFANOV, Anatoly Sergeevich**
**ul. Entuziastov, 1-79**
**Kharkov, 310108 (SU)**

EP 0 403 649 B1

Proprietor: **KONSTANTINOV, Boris Alexeevich**
Severnoe Chertanovo, 4-407-703
Moscow, 113648 (SU)

Proprietor: **FILIMONOV, Valery Nikolaevich**
pr. Mira, 64-4-15
Kirovskaya obl
Kirovo-Chepetsk 613020 (SU)

Proprietor: **BIZJUKOV, Alexandr Vasilievich**
ul. Gagarina, 19-22
Leningradskaya obl
Gatchina, 188350 (SU)

Proprietor: **NELJUBIN, Anatoly Alexeevich**
ul.Zheleznodorozhnaya,6-2-60
Kirovskaya obl
Kirovo-Chepetsk 613020 (SU)

Proprietor: **UDALTSOV, Vladimir Fedorovich**
ul. Rechnaya, 16-72
Kirovskaya obl
Kirovo-Chepetsk 613020 (SU)

Proprietor: **MIKHAILOV, Sergei Vasilievich**
Vyatskaya naberezhnaya, 10-24
Kirovskaya obl
Kirovo-Chepetsk 613020 (SU)

(72) Inventor: **MELNIKOV, Alexandr Petrovich**
ul. Sosnovaya. 22-2-54
Kirovskaya obl
Kirovo-Chepetsk 613020 (SU)
Inventor: **KARTOSHKIN, Vyacheslav Mikhailovich**
pr. Mira, 43e-39
Kirovskaya obl
Kirovo-Chepetsk 613020 (SU)
Inventor: **EVDOKIMOV, Sergei Vasilievich**
ul. Chepetskaya, 20 70
Kirovskaya obl
Kirovo-Chepetsk 613020 (SU)
Inventor: **BROVKO, Miroslav Mikhailovich**

ul. Entuziastov, 5-36
Kharkov, 310108 (SU)
Inventor: **GORSHKOV, Jury Vladimirovich**
pr. Mira, 17-9
Kirovskaya obl
Kirovo-Chepetsk 613020 (SU)
Inventor: **PERIMOV, Jury Alexandrovich**
pr. Mira, 21a-40
Kirovskaya obl
Kirovo-Chepetsk 613020 (SU)
Inventor: **MITROFANOV, Anatoly Sergeevich**
ul. Entuziastov, 1-79
Kharkov, 310108 (SU)
Inventor: **KONSTANTINOV, Boris Alexeevich**
Severnoe Chertanovo, 4-407-703
Moscow, 113648 (SU)
Inventor: **FILIMONOV, Valery Nikolaevich**
pr. Mira, 64-4-15
Kirovskaya obl
Kirovo-Chepetsk 613020 (SU)
Inventor: **BIZJUKOV, Alexandr Vasilievich**
ul. Gagarina, 19-22
Leningradskaya obl
Gatchina, 188350 (SU)
Inventor: **NELJUBIN, Anatoly Alexeevich**
ul.Zheleznodorozhnaya,6-2-60
Kirovskaya obl
Kirovo-Chepetsk 613020 (SU)
Inventor: **UDALTSOV, Vladimir Fedorovich**
ul. Rechnaya, 16-72
Kirovskaya obl
Kirovo-Chepetsk 613020 (SU)
Inventor: **MIKHAILOV, Sergei Vasilievich**
Vyatskaya naberezhnaya, 10-24
Kirovskaya obl
Kirovo-Chepetsk 613020 (SU)

(74) Representative: **Bibby, William Mark et al**
Mathisen, Macara & co.
The Coach House
6-8 Swakeleys Road
Ickenham
Uxbridge UB10 8BZ (GB)

## Description

### Technical Field

The invention relates to medical engineering and, more specifically, to a cardiac valve prosthesis according to the preamble of Claim 1. Such a prosthesis is known from US-A-4 274 437.

The invention can find most utility when applied for replacement of affected natural aortal and mitral cardiac valves in humans. The present invention will meet equal success when used for replacing an affected tricuspid valve.

### Background Art

Cardiovascular diseases have become one of the most powerful enemy of human beings in the last years by virtue of its frequent occurrence and severe complications while treatment of such diseases is one of the most important task of modern surgery.

A cardiac valve prosthesis is essentially a check valve establishing direct blood flow with the valve closing element open and preventing blood reflux (regurgitation) with the closing element occluding the valve orifice.

The problem how to provide cardiac valve prosthesis capable of ensuring even satisfactory replacement of affected cardiac valves has a three-decade long history. However, none of the constructions of cardiac valve prosthesis has so far been engineered, satisfying all requirements of human organism.

It is thrombus formation that occurs to be the most common and threatening complication. To reduce the danger of thrombus formation, it is necessary to eliminate engorgement (hyperemic) zones, provide laminar blood flow, as well as good conditions for the blood to flow past every valve component.

An attempt made to prevent thrombus formation secondary to prosthetic restoration of cardiac valve has resulted in the provision of a cardiac valve prosthesis (US, A, 4,308,624). The valve prosthesis in question has a ring-shaped cage accommodating a closing element associated with the cage through a means for turning said closing element from the closed position into the open one, and vice versa. The closing element is shaped as two arcuate cusps while the concave surface of each of the cusps faces towards blood reflux with the closing element in the closed position.

The inner surface of the valve cage is cylinder-shaped, its portion facing direct blood flow having a smaller diameter than the other portion thereof facing blood reflux. The place where both of the cylindrical-shaped cage portions are jointed together serves as the valve seat. Two diametrally opposite flat portions are provided on the cage inner surface throughout the entire cage height.

The means for turning each of the valve cusp is essentially two interacting elements, one of which being located on the flat portions of the cage inner surface while the other is situated on the opposite sides of the closing element.

The element of the turning means located on the flat portions of the cage inner surface is shaped as two oblong recesses whose axes make up an angle of 20 degrees with the diametral cage plane. The element of the turning means of the valve closing element situated on the opposite sides of the latter is shaped as a spherical projection.

An annular groove is provided on the cage outer surface, said groove being confined within two collars located at the valve inlet and outlet, respectively, as viewed along the direction of direct blood flow, and being adapted to accommodate the cuff for stitching the valve to the cardiac tissues.

During operation of the above discussed cardiac prosthesis, the cusps of the closing element interact, at the instant of the valve closure, with the valve seat, that is, with the cage inner surface at the same points, which results in increased wear on the cage inner surface; moreover there occurs abnormally high wear of the elements of the closing element turning means, i.e., the spherical projections on the cusps and the recesses in the flat portions of the cage inner surfaces. All this affects badly the service life of the cardiac valve prosthesis under consideration.

With the cardiac valve open its cusps assume the same position with respect to the valve central axis throughout the entire service life of the valve prosthesis, whereby hyperemic zones are liable to occur behind the valve cusps, which promote the thrombosis processes; besides, there occurs also distorted geometry of the portions of the cardiac canal walls due to their being under prolonged effect of the blood flow passing through the valve prosthesis, which inflicts complications upon the myocardium.

Since the concave surface of each cusp faces towards blood reflux with the valve closed, the cusps are acted upon by a comparatively low moment of force, according to the laws of aerodynamics, with the result that an incomplete opening of the valve cusps occurs, resulting in increased hydraulic resistance of the valve prosthesis and/or a slowed down closing of the cusps and, hence, in increased regurgitation, which, in turn, affects adversely the hemodynamic valve characteristics.

When in the open position the cusps of the heretofore-known construction of a cardiac valve prosthesis define two narrowing canals, together with the cage inner surface, adapted for the blood to flow along. The result is the converging blood flow and, hence, an increased hydraulic resistance of the valve and an additional load applied to the heart.

Furthermore, provision of two collars on the cage outer surface between which the cuff is secured, is in fact the source of additional stress concentrators, which tells negatively on the reliability of the entire construction of the valve prosthesis and might result in its breakage during installation of the cusps into the valve cage, or in the course of valve operation, as well as results in a greater diameter of the valve seating surface and, hence, in a lower ratio between the diameter (area) of the valve flow section and the diameter (area) of its seating surface, which in turn affects adversely the valve efficacy.

Other cardiac valve prostheses of this kind are described in EP-A-327,790 (representing a prior art according to Art. 54(3) EPC) and US-A-4,274,437.

In the case of the cardiac valve prosthesis described in EP-A-327,790, the turning means for enabling respective parts of the closing element to rotate relative to each other comprises slots formed in opposite end surfaces of the parts and a projection provided on an inner surface of the circular cage and being received in the slots.

It is an object of the invention to provide a cardiac prosthesis valve having improved haemodynamic characteristics and substantially alleviating the aforementioned problems associated with thrombosis formation and poor service life.

According to the invention there is provided a cardiac valve prosthesis comprising, a circular cage having a central axis, a closing element mounted within the circular cage, turning means for enabling respective parts of the closing element to rotate from respective closed to respective open positions, and vice versa, and means for ensuring that the parts are spaced apart from each other in their respective open positions by a preset distance measured in a plane perpendicular to said central axis, and said turning means being provided with a slot and a projection received in the slot characterised in that respective said slots are formed in opposite end surfaces of said parts, said projection is a circular projection formed on, and following the perimeter of an inner surface of the circular cage, said parts are cusp-shaped and said means comprises cam surfaces of the parts which face and contact each other.

Said means for ensuring that the cusp-shaped members are spaced apart from each other in their respective open positions by said preset distance results in a preset amount of the central blood flow being free to pass through said gap, thus improving the haemodynamic characteristics of the cardiac valve prosthesis and creating better conditions for the blood to flow past every surface of the valve components, resulting in a reduced risk of thrombosis. The profiled portions of the contacting cam surfaces cause the valve cusps to part at the initial instant of their opening when the blood pressure applied to the cusps is maximal.

Furthermore, this construction of cardiac valve prosthesis provides for movement of the closing element along the perimeter of the cage inner surface, thus changing the angular position of the closing element at every instant of time, a feature that prevents the onset of hyperemic zones, further reducing the risk of thrombosis. The element of the turning means is shaped circular so as to follow the perimeter of the cage inner surface, thus giving rise to favourable conditions for increasing the service life of the valve prosthesis due to the fact that the contact with the lateral surface of the closing element occurs at different points of the cage inner surface.

In a preferred embodiment of the invention, the sides of the slots are defined by respective pairs of lines that are mutually inclined at obtuse angles, respective first lines of the pairs defining sides that are emergent at surfaces of the cusp-shaped members that face direct blood flow, and respective second lines of the pairs defining sides that are emergent at surfaces of the cusp-shaped members that face blood reflux, wherein the lines that define opposite sides of the slots and that are emergent at opposite surfaces of the cusp-shaped members are parallel, and corresponding lines formed in opposite end surfaces of the cusp-shaped members are of unequal length.

The fact that the element of the turning means located on the inner surface of the ring-shaped cage is shaped as an annular projection and the element of the turning means situated on each of the opposite sides of the lateral surface of the closing element is shaped as an open-ended slot renders the proposed construction of the cardiac valve prosthesis more productionally effective. Moreover, such a construction arrangement of the proposed cardiac valve prosthesis adds to its service life, this being due to the fact that wear on the projection is minimized since its contact with the slot occurs at different points on its surface, while any wear on the slot surface does not affect materially the reliability of the valve prosthesis construction and results in a slight increase in regurgitation. The fact that the slot is of the open-ended design is conducive to blood flow past the slot, thus preventing the onset of hyperemic zones therein, which are causative to thrombosis.

The aforesaid construction arrangement of the slot defines its technological effectiveness, makes it possible to retain a required preset magnitude of the opening angle of the closing element and to provide forced movement of the closing element along the perimeter of the cage inner surface, a feature that prevents arresting of the closing element due to manufacturing imperfections and inaccuracies of the prosthetic valve cage and of the closing element, as well as due to sedimentation of the blood corpuscles, and so on.

Preferably, each cusp-shaped member is defined by an envelope having a concave surface facing direct blood flow and having a radius R of curvature of a median surface according to the inequality $R \leq 10.D_o$, where $D_o$ is the minimum inside diameter of the circular cage.

It is common knowledge that when blood flows past the valve, cusp forces are liable to arise, which define the torque value about the axes of rotation of the valve cusps.

The value of the torque applied to the valve cusps is maximum for their profiles that face with their concave surface the direct blood flow. The aforesaid construction arrangement of the valve cusps provides for a maximum value of the torque applied to the valve cusps, thus causing them to rapidly open for a preset angle.

The aforementioned effect of an increased value of the torque occurs for the valve cusps featuring the radius of curvature of its median surface obeying the following inequality: $R = 10\ D_o$, whereas with $R > 10\ D_o$, the torque applied to the valve cusps increases inconsiderably. With the valve cusps open a gradually diverging channel is defined between the cusps for the blood flow to pass, whereby an inseparable flowing past the valve cusps is attained. The profiles of the valve cusps are responsible for a maximum torque value when the cusps are being closed. As a result, rapid valve closing is attained and the amount of regurgitation is reduced. All this is conducive to better haemodynamic characteristics of the proposed cardiac valve prosthesis.

Preferably, an inner surface of the cage located downstream, with respect to direct blood flow, of said projection is effective as a diffuser having progressively divergent curved walls, and a diffuser expansion angle $\phi$ according to the inequality: $2 . (\theta - \beta - 90°) \leq \phi \leq 2,5 . (\theta - \beta - 90°)$, where $\beta$ is an angle between a straight line interconnecting two points defining the respective ends of a median surface of cusp-shaped member in a section on the diametral plane perpendicular to the axis of rotation in the closed position of the closing element, and of the plane perpendicular to central axis of the valve prosthesis cage; and $\theta$ is an obtuse angle between respective pairs of lines defining the sides of the slots, and an inner surface of the cage located upstream, with respect to direct blood flow, of said projection is progressively convergent.

Such a construction arrangement of the inner surface of the valve prosthesis cage improves the valve haemodynamic characteristics. The fact that the cage inner surface is gradually convergent at the inlet of direct blood flow reduces hydraulic resistance of the valve, while provision of the inner cage surface as a diffuser prevents churning at the outlet of direct blood flow from the valve prosthesis, since the blood flow, when passing over the diffuser walls, acquires apart from the axial velocity component, also the radial component thereof directed towards the walls of the cardiac chamber. Restrictions imposed upon the diffuser expansion angle are dictated by formation of an inseparable stream of direct blood flow through the present valve prosthesis, which renders the danger of hemolysis less probable and reduces the differential pressure on the valve.

Furthermore, an outer surface of the cage may be smoothly concave, and an angle $\gamma$ between a straight line interconnecting the extreme points of the generatrix of the concave surface in a section of the valve cage with the diametral plane and the central axis of the cage is in accordance with the inequality:

$$0{,}4\ \phi \leq \gamma \leq 0{,}6\ \phi,$$

where $\phi$ is the expansion angle of the diffuser of the inner surface of the cage.

Such a construction arrangement of the valve prosthesis adds to its strength characteristics due to the fact that the cage outer surface is smoothly concave, which provides for equal thickness of the cage walls and, hence, reduces the number of stress concentrators. An appropriate relationship between the angle $\phi$ of expansion of the diffuser-shaped cage inner surface and the angle $\gamma$ bound by a straight line interconnecting the extreme points of the generatrix of the cage concave outer surface and the cage central axis makes it possible to select an optimum ratio of the diameter (area) of the valve flow section to the diameter (area) of the valve seating surface, which adds to the valve efficiency and renders its implantation more reliable. With $\gamma \leq 0.6\ \phi$ the efficiency of the valve is increased, while with $\gamma \geq 0.4\ \phi$ the cuff is prevented from slipping off the valve cage outer surface.

A portion of the concave outer surface of the cage may have a retainer having a curvilinear inner surface, the curvature of which corresponds to the curvature of said portion of the concave outer surface of the cage, and an outer surface of substantially frustoconical shape defining an included angle $\psi$ in the range from 10° to 30°.

Provision of the aforesaid rigid retainer on the cage outer surface, said retainer having a curved inner surface, adds to the reliability of the proposed valve prosthesis due to higher rigidity of its cage, which protects the valve against deformation when implanted into human organism.

The fact that the retainer outer surface is shaped as a cone frustum having an included angle ranging within 10 and 30 degrees facilitates introduction of the valve prosthesis into the opening of the fibrous ring during surgery, as well as makes it possible to prevent blood from escaping beyond the cardiac valve prosthesis.

The cardiac valve prosthesis, according to the invention, enables one to reduce the danger of thrombosis, features better haemodynamic characteristics and higher reliability of the valve construction.

Brief Description of the Drawings

The essence of the invention will become more evident from the following specific exemplary embodiments thereof to be had with reference to the accompanying drawings, wherein:

Fig. 1 is a diagrammatic view of human heart showing an aortal and a mitral valve prosthesis implanted thereinto, according to the invention, as well as a fragmentarily cutaway view of the left ventricle;

Fig. 2 is a isometric view of the cardiac valve prosthesis;

Fig. 3 is a scaled-up section taken along the line III-III in Fig. 2 as revolved for a certain angle to bring it in concidence with the plane of the Drawing;

Fig. 4 is a scaled-up view, taken along the arrow A, of the valve prosthesis cusp in Fig. 2, as revolved for a certain angle to bring it in coincidence with the plane of the Drawing;

Fig. 5 is a view of the same valve cusp of Fig. 2, taken along the arrow B;

Fig. 6 is a scaled-up view of the cardiac valve prosthesis, accorcing to the invention, taken as along direct blood flow with the closing element open;

Fig. 7 is a longitudinal sectional view of the cardiac valve prosthesis, according to the invention, with the valve cusps closed;

Fig. 8 is a longitudinal, partly sectional view of the aortal cardiac valve prosthesis; and

Fig. 9 is a longitudinal, partly sectional view of a mitral cardiac valve prosthesis.

Best Mode of Carrying Out the Invention

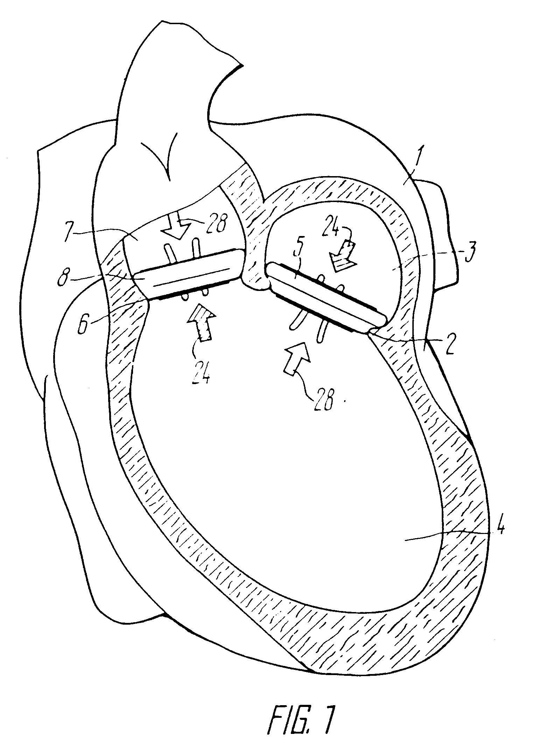

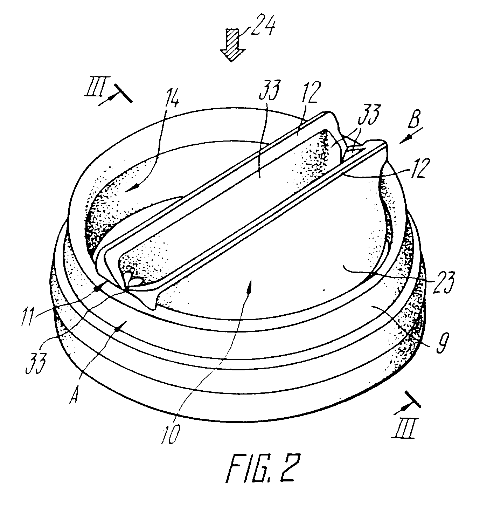

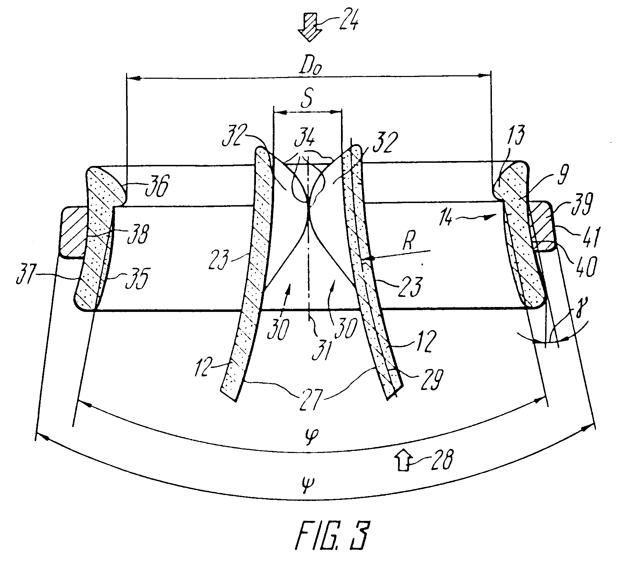

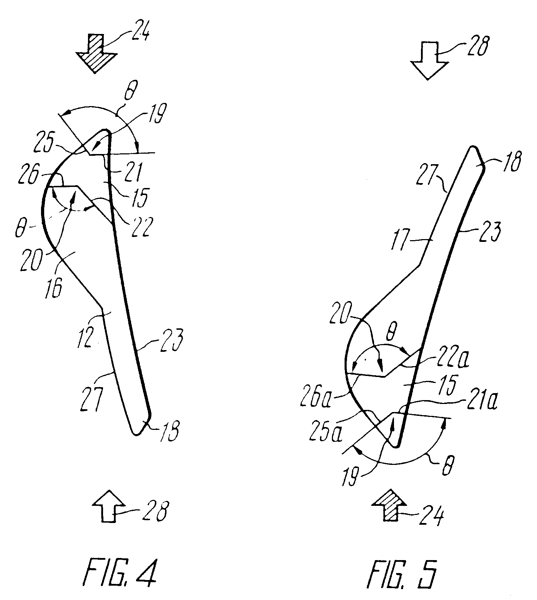

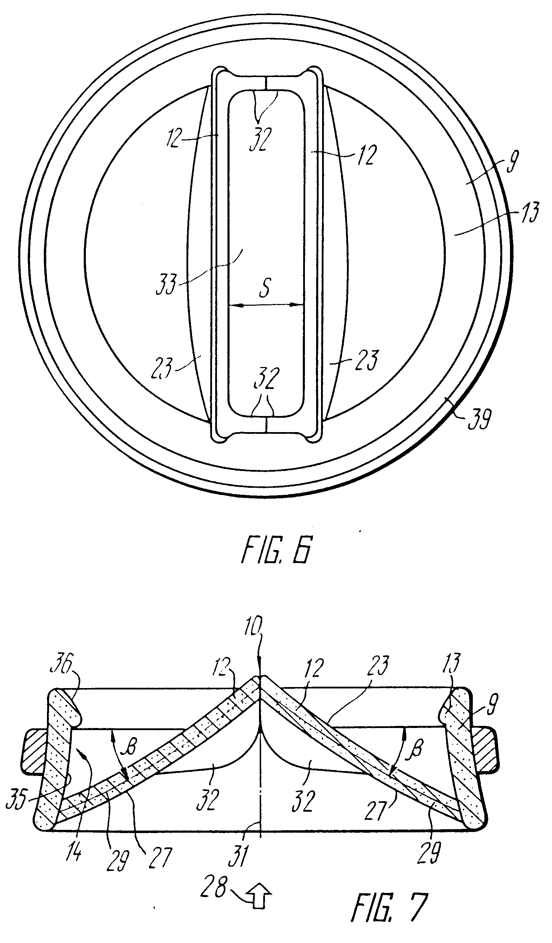

In order to replace affected natural cardiac valves of a heart 1 (Fig. 1), a mitral cardiac valve prosthesis 5 is placed in the opening of a fibrous ring 2, which separates an atrium 3 from a ventricle 4, and an aortal cardiac valve prosthesis 8 is installed in the opening of a fibrous ring 6, which separates the ventricle 4 from an aorta 7. The aortal cardiac valve prosthesis comprises a circular cage 9 (Fig. 2), accommodating a closing element 10 associated with the cage 9 through a means 11 for turning the closing element from the closed to the open position, and vice versa. The closing element 10 is composed of two cusps 12. The means 11 for turning the closing element 10 incorporates two interacting members, that is, a projection 13 (Fig. 3) located on an inner surface 14 of the circular cage 9 and ring-shaped to follow the perimeter of said surface 14, and an open-end slot 15 (Fig. 4) provided on each of the opposite sides 16, 17 (Fig. 5) of a lateral surface 18 of each cusp 12.

The open-end slot 15 provided on the side 16 (Fig. 4) and on the side 17 (Fig. 5) of the lateral surface 18 is confined between two lateral faces appearing as broken lines 19, 20. First portions 21 (Fig. 4) and 22 of the broken lines 19, 20 of the slot 15 provided on the lateral surface 18 on the side 16 thereof, emerge to a concave surface 23 of the cusp 12, which faces the direct blood flow indicated with a hatched arrow 24. Seconds portions 25 and 26 of said broken lines emerge to a surface 27 of the cusp 12, which faced blood reflux indicated with an arrow 28.

The portions 21 and 25, 22 and 26, respectively, of the broken lines 19, 20 make up an obtuse angle $\theta$ confined therebetween, whose magnitude depends on the relation $\theta \leq (180° - \alpha)$, where $\alpha$ is an angle of swivel of the cusp 12 of the closing element 10 from the closed to the open position, or vice versa. The portions 21 and 26, 22 and 25 of the broken lines 19, 20 of the opposite sides of the slot 15 are parallel to each other.

The slot 15 (Fig. 5) provided on the lateral surface 18 on the side 17, is similar to the slot 15 (Fig. 4) made on the lateral surface 18 on the side 16, the sole distinction between said slots being the fact that the like portions 21, 21a (Fig. 5), 22 (Fig. 4) and 22a (Fig. 5), 25 (Fig. 4) and 25a (Fig. 5), 26 (Fig. 4) and 26a (Fig. 5) of the broken lines 19, 20 differ in length.

Each velve cusp 12 (Fig. 3) is in fact part of an envelope having the radius R of curvature of a median 29 complying with an inequality $R \leq 10 \cdot D_o$, where $D_o$ is a minimum inside diameter of the circular valve cage 9. The median surface 29 of the valve cusp 12 is understood to mean such a surface whose points are spaced equidistantly between the surface 23 facing direct blood flow and the surface 27 facing blood reflux. The concave surface 23 of each cusp 12 faces towards direct blood flow. Each of the cusps 12 of the closing element of the cardiac valve prosthesis is provided with a means 30 for establishing a spacing S therebetween preset in the open position of the closing element, said spacing being measured in a plane square with a central axis 31 of the valve prosthesis, said means being shaped as a cam surface 32. The number and arrangement of such cam surfaces may vary. In the hereindiscussed embodiment of the valve prosthesis each of the cusps 12 of the closing element has two cam surfaces 32, which are arranged on the diametrally opposite sides with respect to the central axis 31 of the valve prosthesis, which reduces the danger of cocking of the cusps 12 and leaves a central gap 33 between the cusps free for direct blood flow pass therethrough. The cam surfaces 32 (Fig. 3) of each cusp 12 face and contact each other with their profiled portions 34.

An inner surface 35 of the cage 9 located, as viewed along direct blood flow, past the annular projection 13, is in fact a diffuser having gradually divergent curved walls. The diffuser expansion angle $\phi$ is to be selected proceeding from the relation:

$$2 \cdot (\theta - \beta - 90°) \leq \phi \leq 2.5 \cdot (\theta - \beta - 90°),$$

where $\beta$ (Fig. 7) is an angle between a straight line interconnecting two points which are respectively the ends of the median surface 29 of the cusps 12 in a section with the diametral plane square with the axis of rotation of the cusps into the closed position and with the plane square with the centre line 31 of the valve prosthesis cage 9, and $\theta$ (Fig. 4) is an obtuse angle between the portions 21 and 25, 22 and 26 of the respective broken lines 19 and 20 of the open-end slot 15. An inner surface 36 (Fig. 3) of the cage 9 located, as viewed along direct blood flow, before the annular projection 13 of the closing element, is gradually convergent.

A small diffuser expansion angle $\phi$, provision of a portion of the inner surface of the valve cage as a curved-wall diffuser, as well as an appropriate relationship between the diffuser expansion angle $\phi$ and the angles $\theta$ (Fig. 4) and $\beta$ (Fig. 7) which define the position of the cusps 12 when open that contributes to an inseparable stream of direct blood flow, all this ameliorates the hemodynamic characteristics of the valve by reducing its hydraulic resistance.

An outer surface 37 (Fig. 3) of the cage 9 is smoothly concave so that the angle $\gamma$ between a straight line interconnecting the extreme points of the generatrix of the concave surface 37 in the section of the valve cage 9 with the diametral plane and the central axis 31 of the valve cage 9 is consistent with the following inequality:

$$0.4 \, \phi \leq \gamma \leq 0.6 \, \phi$$

where $\phi$ is the expansion angle of the diffuser-shaped inner surface 35 of the valve cage 9.

A retainer 39 is provided on a portion 38 of the concave outer surface 37 of the cage 9, said retainer having a curved inner surface 40, its curvature corresponding to the curvature of the concave portion 38 of the outer surface 37 of the cage 9. An outer surface 41 of the retainer 39 is shaped as a cone frustum having an included angle $\psi$ ranging from 10 to 30 degrees.

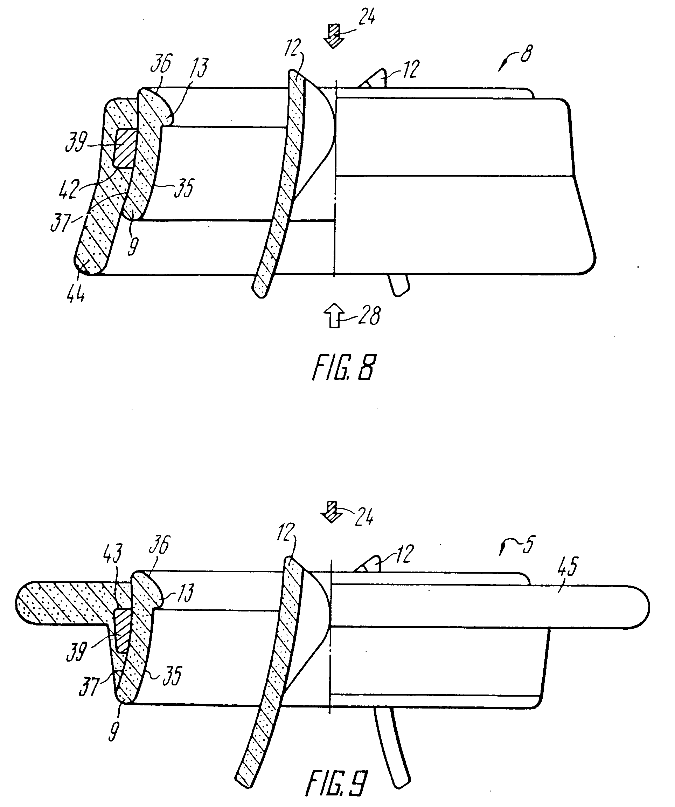

In the aortal cardiac valve prosthesis 8 (Fig. 1) a greater base 42 (Fig. 8) of the cone frustum 39 faces towards blood reflux indicated with the arrow 28.

In the mitral cardiac valve prosthesis 5 (Fig. 1) a greater base 43 (Fig. 9) faces towards direct blood flow indicated with the hatched arrow 24.

A cuff 44 (Fig. 8) is provided, aimed at stitching the aortal cardiac valve prosthesis 8 (Fig. 1) into the fibrous ring 6, said cuff being located on the outer surface 37 of the cage 9 and fixed with threads (omitted in the Drawing) at both ends of the retainer 39.

A cup 45 (Fig. 9) is provided for stitching the mitral cardiac valve prosthesis 5 into the fibrous ring 2, said cuff being situated on the outer surface 37 of the cage 9 and fixed with threads (omitted in the Drawing) at both ends of the retainer 39.

The implantation procedure and surgical techniques of the mitral valve prosthesis 5 and the aortal valve prosthesis 8 are left beyond the scope as being known commonly from numerous publications. It is worth noting only the fact that relationship between the angle $\phi$ (Fig. 3) of expansion of the diffuser-shaped inner surface 35 of the cage 9 and the angle $\gamma$ confined within a straight line interconnecting the extreme points of the generatrix of the concave surface 37 and the central axis 31 of the cage 9 makes it possible to optimize the ratio of the diameter (area) of the valve flow section to the diameter (area) of the valve seating surface, a feature that adds to the valve efficiency, while the fact that the outer surface 41 of the retainer 39 is shaped as a cone frustum having an inclined angle $\psi$ ranging from 10 to 30 degrees facilitates introduction of the valve prosthesis 5 or 8 (Fig. 1) into the opening of the fibrous ring 2 or 6, respectively, during surgery, as well as enables one to prevent blood from escaping beyond the cardiac valve prostheses 5 and 8 during operation.

Operation of the proposed cardiac valve prosthesis will now be discussed with reference to the aortal valve prosthesis 8 (Fig. 1).

Contraction of the ventricle 4 of the heart 1 establishes an excess pressure before the valve prosthesis 8, which urges the cusps 12 (Fig. 2) to open, thus admitting blood to flow to the aorta 7 (Fig. 1). Now let us consider in more detail the instant of opening one of the cusps 12 (Fig. 2) of the closing element 10 of the present cardiac valve prosthesis. When acted upon by an excess pressure applied to the cusp 12 the latter is made to turn, due to interaction of the slot 15 (Fig. 4) and the projection 13 (Fig. 3), round an imaginary axis passing through the point of intersection of the portions 21 and 25 (Fig. 4) of the broken line 19 of the slot 15 provided on the lateral surface 18 on the side 16 and through the point of intersection of the portions 21a (Fig. 5) and 25a of the broken line 19 of the slot 15 made on the lateral surface 18 on the side 17 till the portions 21, 26 (Fig. 4) and the portions 21a, 26a (Fig. 5) are brought in interaction with the projection 13 (Fig. 3), which corresponds to a preset magnitude of the angle $\alpha$ of opening of the cusp 12. Since the portion 25 (Fig. 4) of the slot 15 is shorter than the portion 25a (Fig. 5) of the other slot 15, the points on the lateral surface 18 on the side 16 (Fig. 4) of the cusp 12 will move for a shorter distance circumferentially than the points on the lateral surface 18 on the side 17 (Fig. 5). Thus, the cusp 12 is made to travel along the perimeter of the inner surface 14 (Fig. 3) of the cage 9.

Similar reasons hold true of the other cusp 12. Thus, positive motion of the two cusps 12 along the perimeter of the inner surface 14 of the cage 9 prevents formation of hyperemic zones, thus eliminating the causes of thrombosis. The process of opening of the cusps 12 for a preset angle $\alpha$ is also favoured by higher torque about the axes of rotation of the cusps 12, which is due to the effect of a pressure exerted by direct blood flow (indicated with the arrow 24) on the concave surface 23. When the cusps are being opened the profiled portions 34 of their cam surfaces 32 are brought into interaction, with the result that the gap 33 (Fig. 6) of the necessary amount S is established between the cusps 12, allowing a preset quantity of blood to flow therethrough. Besides, the profiled portions 34 (Fig. 3) of the cam surfaces contacting each other provide for bringing the cusps apart at the initial instant of their opening when the blood pressure applied to the cusps is maximum.

With the valve prosthesis open the maximum amount of blood passes therethrough as a direct flow, which is indicated with the arrow 24. The central direct blood flow, while passing through the gap 33, expands gradually since the cusps 12 establish a smoothly divergent canal, whereby an inseparable stream of blood flow past their surfaces 27 is ensured.

Lateral direct blood flow, while passing through a canal established by the concave surface 23 of the cusp 12 and the inner surface 14 of the cage 9, first is gradually converging, since the portion of the inner surface 36 of the cage 9 situated before the annular projection 13, as viewed along the direction of blood flow, is smoothly convergent, then the blood flow is gradually expanding due to the fact that the portion of the inner surface 35 of the cage 9 is shaped as a diffuser, with the result that the blood flow acquires, apart from an axial velocity component, also a radial component thereof, which is directed towards the walls of the chamber of the heart 1 (Fig. 1), thus preventing churning at the outlet of direct blood flow emerging from the valve prosthesis.

Once the ventricle 4 of the heart 1 has relaxed, an excess blood pressure arises past the valve 8, which pressure urges the cusps 12 (Fig. 7) to rapidly close, which is due to an increased value of the torque resulting from the effect of blood reflux on the convex surfaces 27 as indicated with the arrow 28. Thus, the amount of regurgitation is reduced and the valve is made more hermetically tight.

Now let us consider in detail the instant when one of the cusps 12 (Fig. 2) of the valve prosthesis closing element 10 is closed. When acted upon by an excess pressure exerted by blood reflux on the cusp 12 the latter is made to turn, due to interaction of the slot 15 (Fig. 4) and the projection 13 (Fig. 3), round an imaginary axis passing through the point of interaction of the portions 22 and 26 (Fig. 4) of the broken line 20 of the slot 15 made on the lateral surface 18 on the side 16 and through the point of intersection of the portions 22a and 26a (Fig. 5) of the broken line 20 of the slot 15 provided on the lateral surface 18 on the side 17 till the portions 22 and 25 (Fig. 4) and the portions 22a and 25a (Fig. 5) begin to interact with the projection 13 (Fig. 3). Since the portion 26 (Fig. 4) of the slot 15 is shorter than the portion 26a (Fig. 5) of the other slot 15, the points on the lateral surface 18 on the side 16 (Fig. 4) of the cusp 12 will move for a shorter distance circumferentially than the points on said lateral surface 18 on the side 17 (Fig. 5). Thus, the cusp 12 (Fig. 3) is made to travel along the perimeter of the inner surface 14 of the cage 9. Similar reasoning holds true of the other cusp 12.

The afore discussed operation of the cardiac valve prosthesis 8 is reiterated upon further contractions of the ventricle 4 (Fig. 1) of the heart 1.

Operation of the mitral valve prosthesis 5 is similar to that of the aortal valve prosthesis 8. It is worth noting that when the aortal valve 8 is open the mitral valve 5 is closed, and vice versa.

Industrial Applicability

Laboratory studies have demonstrated a minimum pressure loss on the proposed cardiac valve prosthesis when a direct liquid flow passes therethrough and minimum regurgitation as compared to the know-existing cardiac valve prostheses. Trials on an accelerated test rig within a period of time equivalent to a 30-year service life of the valve implanted into human organism, have demonstrated high longevity and reliability of the proposed prostheses, viz., no cases of their destruction have been observed. Wear on the interacting valve prostheses components is negligible, whereas their hydrodynamic and functional characteristics remain unaffected.

Once the proposed cardiac valve prosthesis had passed comprehensive laboratory tests, some specimens thereof were forwarded for extended clinical trialling, which corroborated high thrombus-resistance and hemodynamic efficiency of the proposed construction of cardiac valve prosthesis when used to replace affected human cardiac valves.

**Claims**

1. A cardiac valve prosthesis comprising,
   a circular cage (9) having a central axis (31), a closing element (10) mounted within the circular cage (9), turning means (11) for enabling respective parts (12) of the closing element (10) to rotate from respective closed to respective open positions, and vice versa, and means (30) for ensuring that the parts (12) are spaced apart from each other in their respective open positions by a preset distance measured in a plane perpendicular to said central axis (31), and said turning means (11) being provided with a slot (15) and a projection (13) received in the slot (15) characterised in that respective said slots (15) are formed in opposite end surfaces (16,17) of said parts (12), said projection (13) is a circular projection formed on, and following the perimeter of an inner surface (14) of the circular cage (9), said parts (12) are cusp-shaped and said means (30) comprises cam surfaces (32) of the parts (12) which face and contact each other.

2. A cardiac valve prosthesis as claimed in claim 1, characterised in that the sides of slots (15) are defined by respective pairs of lines that are mutually inclined at obtuse angles, respective first lines (21,22; 21a,22a) of the pairs defining sides that are emergent at surfaces (23) of the cusp-shaped members that face direct blood flow, and respective second lines (25,26; 25a,26a) of the pairs defining sides that are emergent at surfaces (27) of the cusp-shaped members that face blood reflux, wherein the lines (22,25; 21,26; 22a,25a; 21a,26a) that define opposite sides of slots (15) and that are emergent at opposite surfaces (23,27) of the cusp-shaped members are parallel, and corresponding lines (21,21a; 22,22a; 25,25a and 26,26a) formed in opposite end surfaces (16,17) of the cusp-shaped members are of unequal length.

3. A cardiac valve prosthesis as claimed in claim 1, characterised in that each cusp-shaped member is defined by an envelope having a concave surface (23) facing direct blood flow and having a radius R of curvature of a median surface (29) according to the inequality $R \leq 1OD_o$, where $D_o$ is the minimum inside diameter of the circular cage (9).

4. A cardiac valve prosthesis as claimed in claim 1 or claim 3, characterised in that an inner surface (35) of the cage (9) located downstream, with respect to direct blood flow, of said projection (13) is effective as a diffuser having progressively divergent curved walls, and a diffuser expansion angle $\phi$ according to the inequality: $2 \cdot (\theta - \beta - 90°) \leq \phi \leq 2,5 \cdot (\theta - \beta - 90°)$, where $\beta$ is an angle between a straight line interconnecting two points defining the respective ends of a median surface (29) of cusp-shaped member (12) in a section on the diametral plane perpendicular to the axis of rotation in the closed position of the closing element (10), and of the plane perpendicular to central axis (31) of the valve prosthesis cage (9); and
   $\theta$ is an obtuse angle between respective pairs of lines (21,22;21a,22a) defining the sides of slots (15),
   and an inner surface (36) of the cage (9) located upstream, with respect to direct blood flow, of said projection (13) is progressively convergent.

5. A cardiac valve prosthesis as claimed in claim 4, characterised in that an outer surface (37) of the cage (9) is smoothly concave, and an angle $\gamma$ between a straight line interconnecting the extreme points of the generatrix of the concave surface (37) in a section of the valve cage (9) with the diametral plane and the centre line (31) of the cage (9) is in accordance with the inequality:
   $0,4\phi \leq \gamma \leq 0,6\phi$, where $\phi$ is the expansion angle of the diffuser of the inner surface (35) of the cage (9).

6. A cardiac valve prosthesis as claimed in claim 5, characterised in that a portion (38) of the concave outer surface (37) of the cage (9) has a retainer (39) having a curvilinear inner surface (40), the curvature of which corresponds to the curvature of said portion (38) of the concave outer surface (37) of the cage (9), and an outer surface (41) of substantially frustoconical shape defining an included angle $\psi$ in the range from 10° to 30°.

**Patentansprüche**

1. Herzklappenprothese, die
einen kreisförmigen Käfig (9), der eine zentrale Achse (31), ein Schließelement (10), das innerhalb des kreisförmigen Käfigs (9) befestigt ist, eine Dreheinrichtung (11) zur Ermöglichung für die entsprechenden Teile (12) des Schließelements (10) sich von der jeweiligen geschlossenen zu der jeweiligen offenen Position, und umgekehrt, zu drehen, und eine Einrichtung (30) zum Sicherstellen, daß die Teile (12) zueinander in deren jeweiligen offenen Stellungen um einen vorgegebenen Abstand, der in einer Ebene senkrecht zu der zentrale Achse (31) gemessen wird, beabstandet sind, und wobei die Dreheinrichtung (11) mit einem Schlitz (15) und einem Vorsprung (13), der in dem Schlitz (15) aufgenommen ist, ausgestattet ist, aufweist, dadurch gekennzeichnet, daß die jeweiligen Schlitze (15) in gegenüberliegenden Endoberflächen (16,17) der Teile (12) gebildet sind, daß der Vorsprung (13) ein kreisförmiger Vorsprung ist, der an dem Umfang einer inneren Oberfläche (14) des kreiförmigen Käfigs (9) gebildet ist und diesem folgt, wobei die Teile (12) spitzenförmig sind und die Einrichtung (30) Nockenoberflächen (32) der Teile (12) aufweist, die zueinander hin gerichtet sind und sich berühren.

2. Herzklappenprothese nach Anspruch 1, dadurch gekennzeichnet, daß die Seiten der Schlitze (15) durch jeweilige Paare von Linien festgelegt sind, die gemeinsam unter stumpfen Winkeln geneigt sind, wobei die jeweiligen ersten Linien (21,22; 21a,22a) der Paare Seiten festlegen, die an den Oberflächen (23) der spitzenförmigen Teile austretend sind, die zu der direkten Blutströmung hin gerichtet sind, und entsprechende zweite Linien (25,26;25a,26a) der Paare die Seiten festlegen, die an Oberflächen (27) der spitzenförmigen Teile austreten, die zu dem Blutrückfluß hin gerichtet sind, wobei die Linien (22,25; 21,26; 22a,25a; 21a,26a), die gegenüberliegende Seiten der Schlitze (15) festlegen und die an gegenüberliegenden Oberflächen (23,27) der spitzenförmigen Teile austreten, parallel verlaufen, und entsprechende Linien (21,21a; 22,22a; 25,25a und 26,26a), die in gegenüberliegenden Endoberflächen (16,17) der spitzenförmigen Teile gebildet sind, von ungleicher Länge sind.

3. Herzklappenprothese nach Anspruch 1, dadurch gekennzeichnet, daß jedes spitzenförmige Teil durch eine Einhüllende festgelegt ist, die eine konkave Oberfläche (23) besitzt, die zu der direkten Blutströmung hin gerichtet ist und die einen Radius R einer Krümmung einer Medianoberfläche (29) entsprechend der Ungleichung $R \leq 10D_o$ besitzt, wobei $D_o$ der minimale Innendurchmesser des kreisförmigen Käfigs (9) ist.

4. Herklappenprothese nach Anspruch 1 oder Anspruch 3, dadurch gekennzeichnet, daß die innere Oberfläche (35) des Käfigs (9), die ausströmseitig, und zwar hinsichtlich der direkten Blutströmung, des Vorsprungs (13) angeordnet ist, als ein Diffusor wirksam ist, der progressiv divergent gekrümmte Wände und einen Diffusor-Expansionswinkel $\phi$ entsprechend der Ungleichung aufweist:
$$2 . (\theta - \beta - 90°) \leq \phi \leq 2,5 . (\theta - \beta - 90°),$$
wobei $\beta$ ein Winkel zwischen einer geraden Linie ist, der zwei Punkte miteinander verbindet, die die jeweiligen Enden einer Medianoberfläche (29) des spitzenförmigen Teils (12) in einem Schnitt an der diametralen Ebene senkrecht zu der Drehachse in der geschlossenen Stellung des Schließelements (10) und der Ebene senkrecht zu der zentrale Achse (31) des Klappenprothesenkäfigs (9) miteinander verbindet; und
wobei $\theta$ ein stumpfer Winkel zwischen entsprechenden Paaren von Linien (21,22;21a,22a) ist, die die Seiten der Schlitze (15) festlegen,
und eine innere Oberfläche (26) des Käfigs (9), die anströmseitig hinsichtlich der direkten Blutströmung angeordnet ist, des Vorsprungs (13) progressiv konvergent ist.

5. Herzklappenprothese nach Anspruch 4, dadurch gekennzeichnet, daß eine äußere Oberfläche (37) des Käfigs (9) leicht konkav ist und einen Winkel $\gamma$ zwischen einer geraden Linie, die die Extrempunkte der Generatrix der konkaven Oberfläche (37) in einem Schnitt des Klappenkäfigs (9) mit der diametralen Ebene und der Mittenlinie (31) des Käfigs (9), verbindet, der Ungleichung genügt:

$$0,4 \phi \leq \gamma \leq 0,6 \phi ,$$

wobei $\phi$ der Expansionswinkel des Diffusors der inneren Oberfläche 35 des Käfigs (9) ist.

**6.** Herzklappenprothese nach Anspruch 5, dadurch gekennzeichnet, daß ein Bereich (38) der konkaven, äußeren Oberfläche (37) des Käfigs (9) eine Rückhalteeinrichtung (39) besitzt, die eine krummlinige innere Oberfläche (40), wobei die Krümmung davon der Krümmung des Bereichs (38) der konkaven äußeren Oberfläche (37) des Käfigs (9) entspricht, und eine äußere Oberfläche (41) einer im wesentlichen kegelstumpfförmigen Form besitzt, die einen eingeschlosserten Winkel $\psi$ in dem Bereich von 10° bis 30° festlegt.

**Revendications**

**1.** Prothèse de valvule cardiaque comprenant une cage circulaire (9) présentant un axe central (31), un élément de fermeture (10) monté à l'intérieur de la cage circulaire (9), des moyens de rotation (11) pour permettre à des parties respectives (12) de l'élément de fermeture (10) de tourner d'une position fermée respective à une position ouverte respective et vice versa, et des moyens (30) pour assurer que les parties (12) sont espacées l'une de l'autre dans leurs positions respectives d'ouverture d'une distance préétablie, mesurée dans un plan perpendiculaire à l'axe central (31), les moyens de rotation (11) étant pourvus d'une gorge (15) et d'une saillie (13) reçue dans la gorge (15), caractérisée en ce que les gorges (15) respectives sont formées dans des surfaces d'extrémité opposées (16, 17) desdites parties (12), la saillie (13) étant une saillie circulaire formée sur le périmètre d'une surface interne (14) de la cage circulaire (9) et suivant celui-ci, lesdites parties (12) ayant une forme de cuspide et lesdits moyens (30) comprenant des surfaces de came (32) des parties (12) qui se font face et se touchent l'une l'autre.

**2.** Prothèse de valvule cardiaque suivant la revendication 1, caractérisée en ce que les côtés des gorges (15) sont délimités par des paires correspondantes de lignes qui sont mutuellement inclinées sous des angles obtus, les premières lignes correspondantes (21, 22 ; 21a, 22a) des paires délimitant des côtés qui émergent aux surfaces (23) des éléments en forme de cuspide qui font face au courant sanguin direct, et les secondes lignes correspondantes (25, 26 ; 25a, 26a) des paires délimitant des côtés qui émergent aux surfaces (27) des éléments en forme de cuspide qui font face au reflux sanguin, les lignes (22, 25 ; 21, 26 ; 22a, 25a ; 21a, 26a) qui définissent des côtés opposés des gorges (15) et qui émergent à des surfaces opposées (23, 27) des éléments en forme de cuspide étant parallèles, et les lignes correspondantes (21, 21a ; 22, 22a ; 25, 25a et 26, 26a) formées dans des surfaces d'extrémité opposées (16, 17) des éléments en forme de cuspide étant de longueur inégale.

**3.** Prothèse de valvule cardiaque suivant la revendication 1, caractérisée en ce que chaque élément en forme de cuspide est délimité par une enveloppe présentant une surface concave (23) qui fait face au courant sanguin direct et qui présente un rayon de courbure R d'une surface médiane (29) selon l'inégalité R $\leq$ 10D$_o$, où D$_o$ est le diamètre interne minimum de la cage circulaire (9).

**4.** Prothèse de valvule cardiaque suivant l'une des revendications 1 et 3, caractérisée en ce qu'une surface interne (35) de la cage (9) située en aval, par rapport au courant sanguin direct, de la saillie (13) est efficace comme diffuseur présentant des parois incurvées progressivement divergentes et un angle d'expansion de diffuseur $\phi$ conforme à l'inégalité :
$2 \cdot (\theta - \beta - 90°) \leq \phi \leq 2,5 \cdot (\theta - \beta - 90°)$, où $\beta$ est un angle compris entre une ligne droite reliant deux points délimitant les extrémités respectives d'une surface médiane (29) d'un élément en forme de cuspide (12) dans une section suivant le plan diamétral perpendiculaire à l'axe de rotation dans la position fermée de l'élément de fermeture (10) et le plan perpendiculaire à l'axe central (31) de la cage de prothèse de valvule (9), et
$\theta$ est un angle obtus entre des paires respectives de lignes (21, 22 ; 21a, 22a) délimitant les côtés des gorges (15),
et en ce qu'une surface interne (36) de la cage (9) située en amont, par rapport au courant sanguin direct, de la saillie (13) est progressivement convergente.

**5.** Prothèse de valvule cardiaque suivant la revendication 4, caractérisée en ce qu'une surface externe (37) de la cage (9) est doucement concave et en ce qu'un angle $\gamma$ entre une ligne droite reliant les points extrêmes de la génératrice de la surface concave (37) dans une section de la cage de valvule (9) par le plan diamétral et la ligne centrale (31) de la cage (9) est conforme à l'inégalité :
$0,4 \phi \leq \gamma \leq 0,6 \phi$, où $\phi$ est l'angle d'expansion du diffuseur de la surface interne (35) de la cage (9).

**6.** Prothèse de valvule cardiaque suivant la revendication 5, caractérisée en ce qu'une partie (38) de la surface externe concave (37) de la cage (9) présente un élément de retenue (39) comportant une surface interne curvilinéaire (40), dont la courbure correspondant à la courbure de ladite partie (38) de la surface externe concave (37) de la cage (9), et une surface externe (41) de forme sensiblement tronconique qui délimite un angle inclus $\psi$ de l'ordre de 10° à 30°.

FIG. 1

FIG. 2

FIG. 3

FIG. 4

FIG. 5

EP 0 403 649 B1

FIG. 6

FIG. 7

FIG. 8

FIG. 9